# EUROPEAN PATENT APPLICATION

(11) **EP 1 148 141 A2**
(43) Date of publication of application: **24.10.2001**
(21) Application number: 01104676.0
(22) Date of filing: 24.02.2001
(51) Int. Cl.: C12Q 1/02, C12N 1/04

(54) **Bioluminescent organism for detecting toxic substances**

(30) Priority: 03.03.2000 KR 2000010763
(71) Applicant: Lee, Kyu-Ho, Environmental Science Major Hankuk University of Foreign Studies, Yongin-city, Kyungki-do (KR); Kim, Sang-Jong, Seoul (KR)
(72) Inventor: Lee, Kyu-Ho, Environmental Science Major Hankuk, Yongin-city, Kyungki-do (KR); Park, Kyoung-Je, Sarang-Maeul 1614-1502, Bucheon-city, Kyungki-do (KR); Kim, Sang-Jong, Mokdong Apt. 907-1304, Seoul (KR); Lee, Dong-Hun, Doojin-Baekro Apt. 105-20, Cheongju-city, Choongcheongbuk-do (KR); Jahng, DeokJin, Cheongsol-Maeul 1003-1303, Seongnam-city, Kyungki-do (KR); Cho, Jang-Cheon, Shinbong Apt. 101-1418, Seoul (KR); Ihm, Hyuk-Soon, Suwon-city, Kyungki-do (KR)
(74) Representative: Lorenz, Werner

(57) **Abstract**

The present invention relates to a bioluminescent organism for detecting toxic substances, specifically to a microorganism YH9-RC which shows very sensitively change in the degree of luminescence when it contacts toxic substances, a method for detecting toxic substances using the changes in the degree of luminescence of the microorganism, and a kit for detecting toxic substances. Toxic substances of very low concentration can be detected using the luminescent organism YH9-RC of the present invention.

## Description

### BACKGROUND OF THE INVENTION

### (a) Field of the Invention

The present invention relates to a bioluminescent organism for detecting toxic substances. Specifically, the present invention relates to a microorganism YH9-RC which sensitively shows a change in the degree of luminescence when it is exposed to toxic substances, a method for detecting toxic substances using changes in the degree of luminescence of the microorganism, and a kit for detecting toxic substances

### (b) Description of the Related Art

Toxicity tests are important methods for detecting deleterious substances existing in water and soil. For this purpose, the methods measuring the toxicity from deleterious substances using organisms have been widely used. Since the microorganisms among various organisms are more economical than higher organisms, toxicity test using microorganisms have been studied a great deal.

U.S. Patent No. 4,808,517 disclosed a toxic test method using mitochondria suspension which reduces NAD+ to NADH. However, this method is difficult and complicated in operational procedures because it uses mitochondria suspension.

Although U.S. Patent No. 5,094,944 disclosed a method for determining the degree of toxic substance content in solution using enzymes and higher organisms, this method is also complicated and has a problem in that reaction does not occur with toxic substances of low concentration.

In addition, although U.S. Patent Nos. 5,565,365 and 5,731,163 disclosed a method for detecting toxic substances using bioluminescent organisms, these methods did not show satisfactory sensitivity to toxic substances.

### SUMMARY OF THE INVENTION

Accordingly, in order to solve these problems of the prior art, it is an object of the present invention to provide a microorganism which can exhibit detection capacity for toxic substances of very low concentration.

It is another object of the present invention to provide a method for detecting toxic substances using the microorganism.

It is still another object of the present invention to provide a kit for detecting toxic substances using the microorganisms.

In order to achieve these objects, the present invention provides a bacterial strain YH9-RC deposited under KCTC 0730BP, which reacts with toxic substances to show change in the degree of luminescence

The present invention also provides a method for detecting toxic substances comprising the step of determining the existence and the concentration of toxic substances using microorganisms, characterized in that the microorganism is YH9-RC deposited under KCTC 0730BP, and an aldehyde is added as a substrate so that the microorganism can emit light.

The present invention also provides a toxic substance analyzing kit comprising a toxic substance sample, a microorganism which reacts with the sample and shows change in the degree of luminescence, a reactor where the sample can react with the microorganism, and a light-detection apparatus which can detect light emitted from the microorganism, characterized in that the microorganism is YH9-RC deposited under KCTC 0730BP, and an aldehyde is introduced into the reactor so that the microorganism can emit light.

### BRIEF DESCRIPTION OF THE DRAWINGS

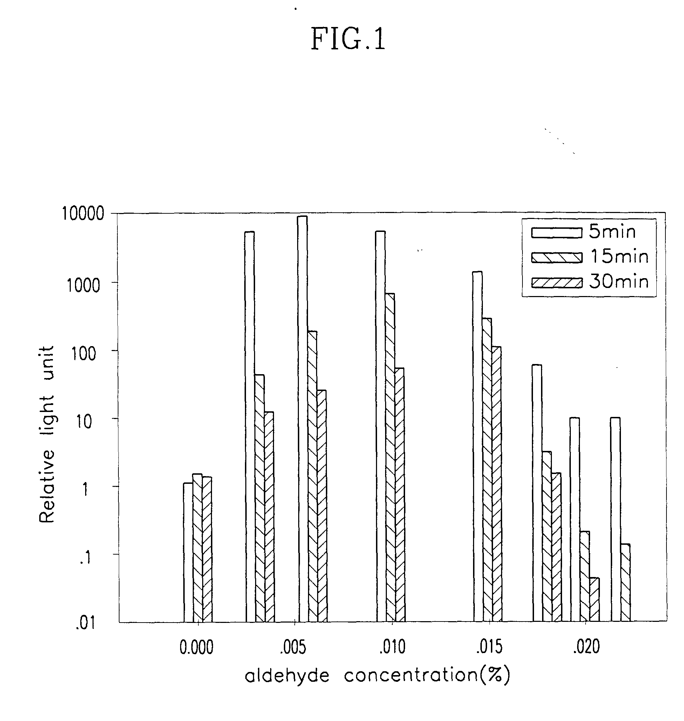

Fig. 1 is a graph showing the optimal concentration of aldehyde (n-decyl) for a toxicity test using YH9-RC.

### DETAILED DESCRIPTION AND THE PREFERRED EMBODIMENTS

The present invention will now be explained in more detail.

The present inventor studied microorganisms which react very sensitively to toxic substances, and as a result of preparing a transformant by introducing genes for luminescence into bacteria and conducting toxicity tests with the microorganism, discovered that the microorganism showed satisfactory sensitivity to toxic substances, and completed the present invention.

The microorganism of the present invention is YH9-RC deposited under KCTC 0730BP. The microorganism (YH9) was isolated from an underground water sample by spreading its aliquot on an R2A agar plate, showed an excellent sensitivity to phenol, and identified to be *Janthinobacterium lividum.*

The introduction of the luminescent genes into the YH9 strain was conducted by introducing the plasmid DNA containing *lux* gene (pUT*luxAB*, Herrero. M.V., V. de Lorenzo, and K. Timmis. 1990. Transposon vectors containing non-antibiotic resistance selection markers for cloning and stable chromosomal insertion of foreign genes in Gram-negative bacteria. J. Bacteriol. 172: 6557-6567) into a recipient cell by a triparental conjugation method. For this purpose, the *lux* gene within the donor strain (*Escherichia coli* S17-1 λ pir strain) was mated with a recipient cell to obtain YH9-RC. As the recipient cell, a spontaneous mutant YH9-R that was modified to have a resistance through standing a stationary phase YH9 cell on an R2A agar plate with 50 *µ*g/ml of rifampicin for 48 hours for ease of the selection of the exconjugant. As a result of examination of the degree of luminescence and preparation of plasmid DNA, it was found that the *lux* gene was stably maintained in a plasmid state and expressed the luminescence function well.

In addition, the present invention provides a method for detecting toxic substances comprising the step of determining the existence and the concentration of toxic substances using a microorganism, wherein the microorganism is YH9-RC KCTC 0730BP, and an aldehyde is added as a substrate so that the microorganism can emit light. Generally, a toxic substance-detecting method for determining the existence and the concentration of a trace amount of toxic substances using a microorganism comprises contacting a microorganism with a sample to be detected, comparing change in the degree of luminescence by the microorganism with that in a control test where a microorganism is not reacted with toxic substances, and converting the decrease in luminescence into a concentration to determine the existence and the concentration of toxic substances. However, the present invention is not limited to the above-mentioned method, and includes any common toxic substance-detecting method using a microorganism wherein the microorganism is YH9-RC. In the toxic substance detecting method using YH9-RC of the present invention, an aldehyde (n-decyl) should be added in order to impart luminescence to the microorganism. The YH9-RC can show the luminescence phenomenon only if a substrate for luciferase, an aldehyde, is added, because *luxA* and *luxB* that code for an essential enzyme, luciferase, are introduced.

Fig. 1 is a graph showing the degree of luminescence measured using a luminometer (Turner Co.) for 30 minutes under the maintained conditions of 23 to 25 °C after YH9-RC is added by aldehyde solutions of various concentrations in order to determine an optimal concentration of aldehyde for a suitable condition of the toxicity test. The results shown in Fig. 1 indicate that the optimum concentration of aldehyde for detecting toxic substances is 0.010 to 0.015 wt%.

In addition, the present invention provides a toxic substance analyzing kit comprising a toxic substance sample, a microorganism which reacts with the toxic sample and shows a change in the degree of luminescence, a reactor where the sample reacts with the microorganism, and a light-detection apparatus which can detect light emitted from the microorganism, wherein the microorganism is YH9-RC deposited under KCTC 0730BP, and aldehyde is introduced into the reactor so that the microorganism can emit light. The toxic substance analyzing kit of the present invention can program the degree of luminescence of YH9-RC according to the kinds and concentrations of toxic substances and cause a sample to react with YH9-RC to determine the concentration of toxic substances in the sample. In addition, toxic substances can be detected more precisely by adding an appropriate number of reactors to the kit to cause a sample to react with YH9-RC, and simultaneously conducting a control test where YH9-RC is not reacted with a toxic sample (for example, detecting the degree of luminescence of YH9-RC which does not react with the sample, in order to react toxic substances of standard concentration with YH9-RC to detect the degree of luminescence). In addition, in the toxic substance analyzing kit of the present invention, the concentration of aldehyde is preferably 0.010 to 0.015 wt%.

The toxic substance detecting method and the toxic substance analyzing kit according to the present invention can be employed for detecting a variety of toxic substances, and' particularly they show excellent sensitivity to organic compounds such as phenol, benzene, toluene, xylene, etc., and heavy metals such as Al, As, Cd, Co, Cr(VI), Cu, Fe, Hg, Mn, Pb, Se, Zn, etc.

The present invention will now be explained with reference to the following Examples. However, these Examples are to illustrate the present invention and the present invention is not limited to them.

### [Example 1]

### Separation and identification of YH9-RC

Among the Gram-negative bacteria selected from a variety of bacterial strains in the water samples of underground water, YH9 exhibits an excellent sensitivity to phenol, which was identified as *Janthinobacterium lividum.* Then, the YH9 was transformed, and luminescent genes were introduced therein.

The following Table 1 presents the minimum inhibitory concentrations (MIC) of the various groundwater bacterial isolates to phenol.

**[Table 1]**

| Bacterial strain | Gram negative | Concentration of Phenol | | | | | | | |
|---|---|---|---|---|---|---|---|---|---|
| | | 0 mM | | 0.5 mM | | 1 mM | | 2.5 mM | |
| | | 24hr | 48hr | 24hr | 48hr | 24hr | 48hr | 24hr | 48hr |
| YH1-1 | - | + | + | + | + | + | + | + | + |
| YH-3 | - | + | + | + | + | + | + | (+) | + |
| YH-4 | - | + | + | + | + | + | + | + | + |
| YH-5 | + | + | + | + | + | + | + | + | + |
| YH-5-1 | - | + | + | + | + | + | + | + | + |
| YH-6 | + | + | + | + | + | + | + | + | + |
| YH-6-1 | Nd | + | + | + | + | + | + | + | + |
| YH-7 | Nd | + | + | + | + | + | + | + | + |
| YH-8 | - | (+) | (+) | (+) | (+) | - | - | - | - |
| YH-9 | - | + | + | + | + | - | (+) | - | - |
| YH-9-2 | - | + | + | + | + | + | + | + | + |
| YH-9-3 | - | + | + | + | + | + | + | + | + |
| YH-10 | - | + | + | + | + | + | + | (+) | + |
| YH-10-1 | - | + | + | + | + | + | + | + | + |
| YH-10-2 | - | + | + | + | + | + | + | + | + |
| YH-11 | - | + | + | + | + | - | + | - | + |
| YH-11-1 | - | + | + | + | + | + | + | (+) | (+) |
| YH-12 | - | (+) | + | (+) | + | (+) | + | (+) | + |
| YH-13 | - | + | + | + | + | (+) | (+) | - | - |
| YH-13-1 | - | + | + | + | + | + | + | - | - |
| YH-13-2 | - | + | + | + | + | + | + | - | - |
| YH-13-3 | - | + | + | + | + | + | + | - | - |
| YH-13-5 | - | + | + | + | + | + | + | - | - |
| YH-13-6 | - | + | + | + | + | + | + | - | - |
| YH-13-7 | - | + | + | + | + | + | + | + | - |
| YH-13-8 | - | (+) | + | - | (+) | - | - | - | - |
| YH-13-9 | - | + | + | + | + | + | + | - | - |
| YH-13-11 | - | + | + | + | + | + | + | - | - |
| UV2 | - | + | + | + | + | + | + | + | + |

The results of the Table 1 show that the YH9 strain has the lowest MIC for phenol among various Gram-negative isolates.

Plasmid DNA comprising *lux* genes was introduced into the YH9 strain by a triparental conjugation method. For this purpose, a donor strain of *lux* gene (*Escherichia coli* S17-1 λ pir strain) was conjugated with a recipient cell to obtain YH9-RC. As the recipient cell, a spontaneous mutant YH9-R which was modified to have resistance, after a stationary phase culture of YH9 stood for 48 hours on an R2A agar plate in which 50 *µ*g/ml of rifampicin was added for the ease of selection of the exconjugant, was used. In addition, the luminescent strain was deposited under KCTC 0730BP.

### [Example 2]

Toxicity tests for a variety of substances described in the following Table 2 were conducted using YH9-RC deposited under KCTC 0730BP obtained in the Example 1 to calculate EC₅₀ (Effective concentration 50: the effective concentration of tested organism that decrease luminescence by 50%). It was compared with the EC₅₀ value using oceanic luminescent bacterium (*Vibrio* sp. Isolate) that was most commonly used in a conventional toxicity test. First, the YH9-RC colony was cultured in an R2A broth containing 5 *µ*g/ml of tetracycline (liquid culture medium containing Bacto yeast extract 0.5 wt%, Bacto proteose peptone No. 3 0.5 wt%, Bacto casamino acids 0.5 wt%, Bacto dextrose 0.5 wt%, soluble starch 0.5 wt%, sodium pyruvate 0.5 wt%, potassium phosphate dibasic 0.5 wt%, and magnesium sulfate 0.5 wt%) for 18 hours or more, and then it was sub-cultured in an R2A broth for another 7 to 8 hours, and the aliquot of the fresh culture was used in the toxicity test. In addition, 80 *µℓ* of the cell culture liquid, 80 *µ*ℓ of 0.03 wt% aldehyde aqueous solution, and 40 *µ*ℓ of a toxic sample diluted to various concentrations were mixed in a test tube of a luminometer, and the degrees of luminescence were measured after 5 minutes, 15 minutes, and 30 minutes to calculate EC₅₀.

**[Table 2]**

| | 5 minutes | | 10 minutes | | 15 minutes | |
|---|---|---|---|---|---|---|
| | *Vibrio* | YH9-RC | *Vibrio* | YH9-RC | *Vibrio* | YH9-RC |
| Phenol | 115.6 | 26.5 | 105.1 | 12.0 | 63.1 | 12.7 |
| Benzene | 477.8 | 75.17 | 606.8 | 50.94 | 747.2 | 35.06 |
| Toluene | 87.1 | 5.61 | 120.2 | 5.65 | 142.1 | 6.3 |
| *p*-xylene | 27.4 | 45.33 | 105.1 | 37.74 | 63.1 | 49.8 |
| Cu | 67.3 | 10.1 | 56.5 | 9.5 | 51.1 | 10.5 |
| Pb | 203.9 | 11.8 | 180.0 | 5.8 | 207.3 | Nd |
| Al | 46.1 | 0.93 | 45.8 | Nd | 41.5 | Nd |
| As | 65.6 | 28.4 | 74.1 | 9.3 | 64.4 | 4.3 |
| Cd | 71.4 | 4.62 | 56.1 | 1.81 | 41.4 | 1.10 |
| Co | 71.6 | 1.11 | 54.4 | 1.84 | 41.4 | 1.96 |
| Cr(VI) | 10.3 | 5.88 | 10.4 | 7.53 | 8.8 | 6.29 |
| Fe | 55.1 | 6.69 | 54.3 | 9.22 | 51.3 | 11.0 |
| Hg | 3.5 | 0.31 | 3.1 | 0.22 | 3.1 | 0.19 |
| Mn | 336.6 | 25.0 | 747.1 | Nd | 300.8 | Nd |
| Se | 90.3 | 29.5 | 81.6 | Nd | 70.5 | 52.6 |
| Zn | 41.6 | 1.30 | 39.8 | 1.34 | 38.8 | 1.29 |

The results of the Table 2 show that the luminescent organism YH9-RC of the present invention exhibits an average 10 times or more sensitivity compared to other luminescent strains widely used in the art.

As explained, the bioluminescent organism YH9-RC deposited under KCTC 0730BP has 10 times or more sensitivity compared to other microorganisms that have been used in conventional toxicity tests, and very low concentration of toxic substances can be detected using the bioluminescent strain of the present invention.

## Claims

1. YH9-RC deposited under KCTC 0730BP reacts with toxic substances to show change in the degree of luminescence.

2. A method for detecting toxic substances comprising the step of determining the existence and the concentration of toxic substances using a microorganism, wherein the microorganism is YH9-RC deposited under KCTC 0730BP, and an aldehyde is added as a substrate so that the microorganism can emit light.

3. The method for detecting toxic substances according to claim 2, wherein the concentration of the aldehyde is 0.010 to 0.015 wt%.

4. The method for detecting toxic substances according to claim 2, wherein the toxic substances are selected from the group consisting of phenol, benzene, toluene, xylene, Al, As, Cd, Co, Cr(Vl), Cu, Fe, Hg, Mn, Pb, Se, Zn, and a mixture thereof.

5. A kit for analyzing toxic substances comprising a toxic substance sample, a microorganism which reacts with the toxic substance to show change in the degree of luminescence, a reactor where the sample reacts with the microorganism, and a light-detection apparatus which can detect light emitted from the microorganism, wherein the microorganism is YH9-RC deposited under KCTC 0730BP, and an aldehyde is introduced into the reactor so that the microorganism can emit light.

6. The kit for analyzing toxic substances according to claim 5, wherein the concentration of aldehyde is 0.010 to 0.015 wt%.
